# EUROPÄISCHE PATENTSCHRIFT

(11) **EP 0 688 763 B1**
(45) Veröffentlichungstag und Bekanntmachung des Hinweises auf die Patenterteilung: **08.04.1998**
(21) Anmeldenummer: 95109415.0
(22) Anmeldetag: 19.06.1995
(51) Int. Cl.: C07C 235/16, C07C 231/12

(54) **Verfahren zur Herstellung von Hydroxycarbonsäureaniliden**
Process for preparing anilides of hydroxycarboxylic acids
Procédé pour la préparation d'anilides des acides hydroxycarboxyliques

(30) Priorität: 23.06.1994 DE 4421884
(43) Veröffentlichungstag der Anmeldung: 27.12.1995
(73) Patentinhaber: Clariant GmbH, 65929 Frankfurt am Main (DE)
(72) Erfinder: Dierdorf, Andreas, Dr., D-60529 Frankfurt am Main (DE); Planker, Siegfried, Dr., D-61462 Königstein (DE); Papenfuhs, Theodor, Dr., D-60433 Frankfurt am Main (DE)

(56) Entgegenhaltungen:
- EP-A- 0 008 096
- EP-A- 0 064 486
- DE-A- 2 904 490
- DE-A- 3 038 598
- DE-A- 3 539 394
- US-A- 4 440 780
- US-A- 4 500 740

## Beschreibung

Die vorliegende Erfindung betrifft ein neues, gegenüber dem Stand der Technik verbessertes Verfahren zur Herstellung von Hydroxycarbonsäureaniliden, ausgehend von den entsprechenden Halogencarbonsäureaniliden.

Hydroxycarbonsäureanilide, insbesondere Glykolsäureanilide, stellen eine bedeutsame Stoffgruppe dar und dienen als wichtige Vorprodukte zur Herstellung von Herbiziden (EP-A-300 344) und pharmazeutischen Wirkstoffen (EP-A-284 338, EP-A-363 284) ebenso wie für die Herstellung von Fungiziden (US-4,440,780).

Aufgrund der Bedeutung dieser Stoffgruppe hat es in der Vergangenheit nicht an Versuchen gefehlt, Hydroxycarbonsäureamide und insbesondere Hydroxycarbonsäureanilide auf unterschiedlichen Wegen zugänglich zu machen.

So geht aus der DE-OS-3 038 598 ein Verfahren zur Herstellung von α-Hydroxycarbonsäureamiden hervor, indem man α-Oxycarbonsäureamide, insbesondere die entsprechenden Formyloxyverbindungen mit Alkoholen in Gegenwart katalytischer Mengen von Alkali- oder Erdalkali-Hydroxiden, Hydrogencarbonaten oder Carbonaten umsetzt. Infolge der dabei ablaufenden Umesterung bilden sich die entsprechenden α-Hydroxycarbonsäureamide. Da die für die Umsetzung benötigten α-Oxy-carbonsäureamide in einem separaten Schritt durch Reaktion von α-Chlorcarbonsäureamiden mit Alkalimetallformiaten hergestellt werden müssen, handelt es sich bei der Herstellung der α-Hydroxycarbonsäureamide α-Chlorcarbonsäureamiden- ausgehend von den entsprechenden - in Wirklichkeit um ein zweistufiges Verfahren, das zudem noch den Nachteil aufweist, daß die Herstellung der α-Oxy-carbonsäureamide in Gegenwart eines quartären Ammoniumsalzes durchgeführt wird. Es ist jedoch bekannt, daß derartige quartäre Ammoniumsalze zu Problemen in der Abwasseraufbereitung führen.

Ein weiteres Verfahren zur Herstellung von α-Hydroxycarbonsäureamiden ist der DE-OS-2 904 490 zu entnehmen. Hierbei setzt man in einer ersten Stufe α-Halogencarbonsäureamide mit einem Alkali- oder Erdalkaliacetat in Gegenwart eines quartären Ammoniumsalzes und gegebenenfalls unter Verwendung eines Verdünnungsmittels zu den entsprechenden α-Acetoxycarbonsäureamiden um und entacyliert die α-Acetoxycarbonsäureamide durch Umsetzung mit einem Alkohol in Gegenwart katalytischer Mengen eines Alkali- oder Erdalkalihydroxides oder eines Alkali- oder Erdalkalicarbonates. Bei diesem Verfahren handelt es sich ebenfalls um einen zweistufigen Prozess, bei dem die Verwendung quartärer Ammoniumsalze ebenfalls zu einer unerwünschten Belastung des Abwassers führt.

Die DE-OS-3 539 394 betrifft ebenfalls ein zweistufiges Verfahren zur Herstellung von Glykolsäureamiden, indem man Chloracetamide mit Kaliumcarbonat in Gegenwart eines aprotischen Amids als Verdünnungsmittel und gegebenenfalls in Gegenwart eines Phasentransferkatalysators zu symmetrischen Carbonaten umsetzt und diese entweder nach vorheriger Isolierung in einer separaten zweiten Stufe oder ohne intermediäre Isolierung direkt durch Umsetzung mit einem primären Alkohol durch Umesterung in Gegenwart eines Alkalimetallhydroxids entacyliert. In allen Beispielen wird jedoch in Gegenwart eines Phasentransfer-Katalysators gearbeitet. Außerdem lassen die zum Teil recht niedrigen Ausbeuten (22 bis 80 %) noch Wünsche offen.

Die vorstehend geschilderten Verfahren erfordern einen relativ großen Aufwand, da sie die gewünschten Hydroxycarbonsäureamide über zwei separate, nacheinander ablaufende Reaktionsschritte zugänglich machen. Zudem führen die als Phasentransfer-Katalysatoren verwendeten quartären Ammoniumsalze zu Problemen bei den im Verlauf der Umsetzung anfallenden Abfallprodukten. Sie sind insbesondere aufgrund ihrer ungünstigen Eigenschaften im Abwasser unerwünscht.

Im Hinblick auf die Bedeutung von Hydroxycarbonsäureaniliden stellt es eine Iohnende Aufgabe dar, ein Verfahren zur Herstellung von Hydroxycarbonsäureaniliden bereitzustellen, das die Nachteile der vorstehend genannten Verfahren vermeidet, sich auf einfache Weise und unter Verwendung leicht zugänglicher Ausgangsstoffe und Hilfsstoffe durchführen läßt und darüberhinaus zu einer Verringerung von Abfallprodukten führt.

Gelöst wird diese Aufgabe durch ein Verfahren zur Herstellung von Hydroxycarbonsäureaniliden der allgemeinen Formel (1) 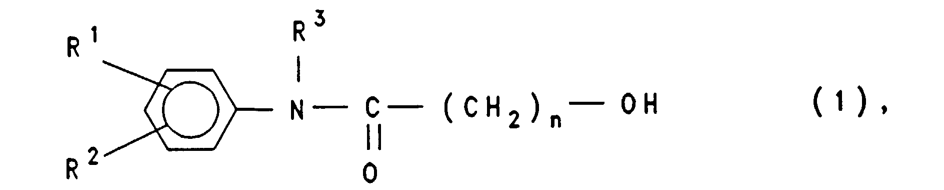 worin R¹ und R² gleich oder verschieden sind und für Wasserstoff, Halogen, eine Nitro-, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, R³ Wasserstoff oder eine geradkettige oder verzweigte Alkyl-Gruppe mit 1 bis 12 Kohlenstoffatomen bedeutet und n für eine ganze Zahl von 1 bis 12 steht. Es ist dadurch gekennzeichnet, daß man ein Halogencarbonsäureanilid der allgemeinen Formel (2) 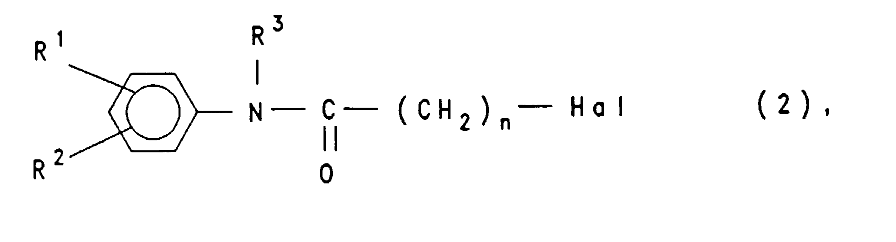 worin R¹, R², R³ und n diesselbe Bedeutung wie in Formel (1) haben und Hal für Chlor, Brom oder Iod steht, mit einer basischen Verbindung in einem Wasser und ein oder mehrere polar aprotische Solventien enthaltenden Lösungsmittelgemisch bei einer Temperatur von 40 bis 180°C umsetzt.

Das erfindungsgemäße Verfahren weist mehrere Vorteile auf. Zum einen führt es in einer einzigen Reaktionsstufe zu dem gewünschten Hydroxycarbonsäureanilid und zum anderen kann auf die Verwendung von Phasentransferkatalysatoren generell verzichtet werden. Darüber hinaus erfordert das erfindungsgemäße Verfahren vergleichsweise kurze Reaktionszeiten und macht die gewünschten Wertprodukte in hohen Ausbeuten und zugleich hoher Reinheit zugänglich. Es läßt sich ohne großen technischen Aufwand und unter Verwendung leicht zugänglicher Ausgangsprodukte realisieren.

Ein weiterer Vorteil besteht darin, daß das aus Wasser und einem oder mehreren polaren aprotischen Solventien bestehende Lösungsmittelgemisch nach erfolgter Umsetzung destillativ entfernt werden kann und sich in die Reaktion wieder einsetzen läßt. Die Anzahl und Menge der Abfallprodukte wird reduziert, da bei dieser Umsetzung als einziges Abfallprodukt lediglich ein Halogenid entsteht.

Es ist als sehr überraschend anzusehen, daß die Umsetzung nicht oder nur in begrenztem Umfange zur Bildung unerwünschter Nebenprodukte führt. Insbesondere war die Bildung von Ethern, gebildet durch Umsetzung von bereits gebildeten Hydroxycarbonsäureaniliden mit noch vorhandenen Halogencarbonsäureaniliden in Gegenwart von basischen Verbindungen, zu erwarten. Überraschenderweise läßt sich die Bildung dieser Ether bei geeigneter Verfahrensführung fast völlig oder weitgehend unterbinden.

Die Reaktion verläuft nach folgender Gleichung

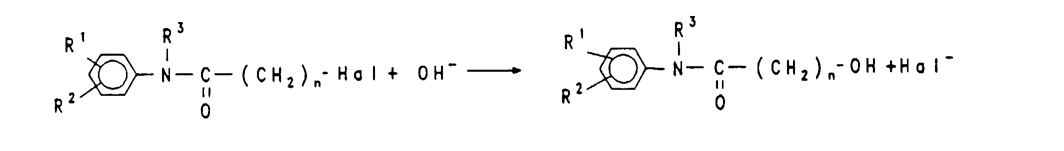

Die für die Umsetzung benötigten Halogencarbonsäureanilide lassen sich mit einem vergleichsweise geringen Aufwand herstellen, indem man ein ω-Halogencarbonsäurechlorid oder ein ω-Halogencarbonsäurebromid mit einem die Reste R¹, R² und R³ enthaltenden Anilin umsetzt. Besonders leicht sind die Chloressigsäureanilide zugänglich, die durch Umsetzung von Chloracetylchlorid mit dem entsprechenden Anilinderivat erhalten werden.

Ohne Anspruch auf Vollständigkeit zu erheben, seien als Beispiele für geeignete Aniline 2-Methoxyanilin, 4-Methoxyanilin, 3,5-Dimethylanilin, 2-Chloranilin, 4-Chloranilin, N-Methylanilin, N-Ethylanilin, N-lsopropylanilin und N-lsopropyl-4-fluoranilin und als Beispiele für geeignete ω-Halogencarbonsäurehalogenide, Chloressigsäurechlorid, Chloressigsäurebromid, ω-Chlorpropionsäurechlorid, ω-Chlorpropionsäurebromid und ω-Bromvaleriansäurechlorid genannt.

Man verwendet für das erfindungsgemäße Verfahren ein Halogencarbonsäureanilid der Formel (2), worin R¹, R², R³, n und Hal die vorstehend genannte Bedeutung besitzen.

Mit gutem Erfolg läßt sich ein Halogencarbonsäureanilid der Formel (2), worin R¹ und R² gleich oder verschieden sind und für Wasserstoff, Halogen, eine Nitro-, eine geradkettige oder verzweigte Alkyl-, Alkoxy-Gruppe mit 1 bis 4 Kohlenstoffatomen oder eine Aralkyl-Gruppe mit 7 bis 12 Kohlenstoffatomen, insbesondere für Wasserstoff, Fluor, Chlor, Brom, eine Alkyl- oder Alkoxy-Gruppe mit 1 bis 4 Kohlenstoffatomen, bevorzugt für Wasserstoff, Fluor, Chlor, Brom oder eine Alkyl-Gruppe mit 1 bis 4 Kohlenstoffatomen steht, einsetzen.

Ebenfalls läßt sich mit gutem Erfolg ein Halogencarbonsäureanilid der Formel (2), worin R³- unabhängig von der jeweiligen Bedeutung von R¹ und R² - für Wasserstoff oder eine geradkettige oder verzweigte Alkylgruppe mit 1 bis 4 Kohlenstoffatomen, insbesondere für eine Isopropylgruppe steht, einsetzen.

Wie eingangs erwähnt, steht n für eine ganze Zahl von 1 bis 12, jedoch insbesondere für eine ganze Zahl von 1 bis 4, bevorzugt für 1. Hal bedeutet, wie zuvor bereits erläutert, Chlor, Brom oder Iod, jedoch insbesondere Chlor oder Brom, bevorzugt Chlor.

Als eine Auswahl einiger geeigneter ω-Halogencarbonsäureanilide seien beispielsweise Chloressigsäure-2-methoxyanilid, Chloressigsäure-4-methoxyanilid, Chloressigsäure-3,5-dimethylanilid, Chloressigsäure-N-isopropyl-4-fluoranilid, Chloressigsäure-N-methylanilid, ω-Bromvaleriansäure-2-chloranilid und ω-Bromvaleriansäure-4-chloranilid genannt.

Als basische Verbindung eignen sich generell alle Substanzen, die in dem aus Wasser und dem polar, aprotischen Lösungsmittel gebildeten Lösungsmittelgemisch Hydroxid-Ionen freisetzen. Hierunter fallen Metallhydroxide, insbesondere Alkalihydroxide und Erdalkalihydroxide, basische Salze, insbesondere Alkalihydrogencarbonate, Erdalkalihydrogencarbonate, Alkalicarbonate, Erdalkalicarbonate, Alkalicarboxylate, Erdalkalicarboxylate, insbesondere Alkali- und Erdalkalisalze von Carbonsäuren mit 1 bis 6 Kohlenstoffatomen, insbesondere von aliphatischen Mono- oder Dicarbonsäuren mit 1 bis 4 Kohlenstoffatomen und Mischungen der vorstehend genannten Stoffe.

Besonders geeignet sind Natriumcarbonat, Kaliumcarbonat, Natriumacetat, insbesondere Natriumcarbonat oder Kaliumcarbonat.

Zur Herstellung des Lösungsmittelgemisches kann man das Gewichtsverhältnis Wasser zu polar, aprotischem Solvens in einem vergleichsweise breiten Bereich einstellen. In den meisten Fällen genügt es, Wasser und polar, aprotisches Solvens in einem Gewichtsverhältnis von 1:4 bis 4:1, insbesondere 1:2 bis 2:1 einzusetzen.

Ohne Anspruch auf Vollständigkeit zu erheben, lassen sich beispielsweise N,N-Dimethylformamid, N,N-Dimethylacetamid, Formamid, Tetrahydrofuran, Dioxan, Dimethylsulfoxid, N-Methylpiperidon oder N-Methylpyrrolidon oder Mischungen derselben, insbesondere N,N-Dimethylacetamid oder N-Methylpyrrolidon als polar, aprotisches Solvens einsetzen.

Obwohl sich die Umsetzung auch mit einem stöchiometrischen Unterschuß an basischer Verbindung, bezogen auf Halogencarbonsäureanilid, durchführen läßt, wird man aus Gründen der Wirtschaftlichkeit basische Verbindung und Halogencarbonsäureanilid in stöchiometrischem Verhältnis oder im Überschuß verwenden. Üblicherweise setzt man je Mol Halogencarbonsäureanilid 1 bis 5 Äquivalente der basischen Verbindung ein. Häufig genügt es, je Mol Halogencarbonsäureanilid 1 bis 1,25 Äquivalente der basischen Verbindung einzusetzen.

Wie eingangs erwähnt, führt man die Reaktion üblicherweise bei 40 bis 180°C durch. Es hat sich zumeist als ausreichend erwiesen, das Halogencarbonsäureanilid bei 60 bis 140, insbesondere 70 bis 120°C umzusetzen. Man arbeitet bei Normaldruck oder bei dem sich jeweils unter den Reaktionsbedingungen einstellenden Reaktionsdruck.

Nach Beendigung der Umsetzung entfernt man üblicherweise das aus Wasser und dem polar aprotischen Solvens respektive Solventien bestehende Lösungsmittelgemisch durch Destillation, gegebenenfalls unter reduziertem Druck, und destilliert den dabei anfallenden Rückstand unter Hochvakuum.

Man kann aber auch auf die Destillation unter Hochvakuum verzichten und den nach Abtrennung des Lösungsmittelgemisches anfallenden Rückstand mit einem geeigneten organischen Lösungsmittel, gegebenenfalls zusammen mit Wasser, extrahieren, die das gewünschte Wertprodukt enthaltende organische Phase mit Wasser waschen, die wäßrige Phase abtrennen und die organische Phase anschließend trocknen.

Als organisches Lösungsmittel eignen sich unter anderem chlorierte aliphatische oder aromatische Kohlenwasserstoffe, beispielsweise Methylenchlorid, Dichlorethan, Chloroform, Chlorbenzol, Dichlorbenzol, Chlortoluol oder aromatische Kohlenwasserstoffe, beispielsweise Toluol, o-Xylol, m-Xylol, p-Xylol, Gemisch isomerer Xylole, Ethylbenzol, Mesitylen. Es lassen sich auch Gemische verschiedener, vorstehend genannter Lösungsmittel verwenden. Besonders geeignet sind Methylenchlorid, Toluol, Chloroform, o-Xylol, m-Xylol, p-Xylol oder Gemische isomerer Xylole.

Die weitere Reinigung des in dem organischen Lösungsmittel vorliegenden Rückstandes erfolgt, gegebenenfalls nach Einengen der organischen Phase, durch Kristallisation.

Die nachfolgenden Beispiele beschreiben die Erfindung, ohne sie zu beschränken.

### Experimenteller Teil

### Vergleichsbeispiele 1 und 2 und Beispiele 1 und 2

### Herstellung von N-Hydroxyacetyl-N-isopropyl-(4-fluoranilin)

23,0 g (0,1 mol) N-Chloracetyl-N-isopropyl-4-fluoranilin werden mit 11,7 g (0,11 mol) Natriumcarbonat in 340 ml Lösungsmittel oder Lösungsmittelgemisch unter Rühren in einem 500 ml Kolben auf Rückflußtemperatur (100°C) erhitzt. Die Umsetzung wird gaschromatographisch verfolgt (Probenahme nach jeweils 1 Stunde).
Die Ergebnisse sind in der nachfolgenden Tabelle zusammengestellt.

**Tabelle:**

| | Lösungsmittel | Lösungsmittelgemisch | Ausbeute* (nach 1 Std. Reaktionszeit) |
|---|---|---|---|
| Vergleichsbeispiel 1 | 340 ml Wasser | - | 29,4 % |
| Vergleichsbeispiel 2 | 340 ml N-Methylpyrrolidon | - | 1,7 % |
| Beispiel 1 | - | 200 ml N-Methylpyrrolidon + 140 ml Wasser | 84,7 % |
| Beispiel 2 | - | 200 ml N,N-Dimethylacetamid + 140 ml Wasser | 73,2 % |

| | | | |
|---|---|---|---|
| *) N-Hydroxyacetyl-N-isopropyl-(4-fluoranilin) bestimmt durch GC-Analyse des Reaktionsgemisches) | | | |

Wie die vorstehenden Ergebnisse belegen, führt das erfindungsgemäße Verfahren (Beispiele 1 und 2) zu signifikant besseren Resultaten als eine Arbeitsweise unter Verwendung von Wasser, jedoch ohne polar aprotisches Solvens (Vergleichsversuch 1), oder als eine Arbeitsweise unter Verwendung eines polar aprotischen Solvens (N-Methylpyrrolidon), jedoch ohne Wasser (Vergleichsbeispiel 2).

### Beispiel 3

### Herstellung von N-Hydroxyacetyl-N-isopropyl-(4-fluoranilin)

In einer entsprechenden Apparatur (500 ml-Kolben) werden 23,0 g (0,1 mol) N-Chloracetyl-N-isopropyl-4-fluoranilin mit 11,7 g (0,11 mol) Natriumcarbonat in einer Lösung aus 140 ml Wasser und 200 ml N-Methylpyrrolidon unter Rückfluß (100°C) erhitzt. Nach 2,5 Stunden wird das Lösungsmittelgemisch im Vakuum abgezogen und der Rückstand bei 2 bis 3 Torr und 132 bis 135°C destilliert. Man erhält 18,1 g (86 % d. Th.) N-Hydroxyacetyl-N-isopropyl-(4-fluoranilin) mit einer Reinheit von 99,1 % (GC).

### Beispiel 4

### Herstellung von N-Hydroxyacetyl-(3,5-dimethylanilid)

In einem 1l-Kolben werden 39,5 g (0,2 mol) Chloressigsäure-(3,5-dimethylanilid) (hergestellt aus Chloracetylchlorid und 3,5-Dimethylanilin) mit 23,3 g (0,22 mol) Natriumcarbonat in einem Lösungsmittelgemisch aus 450 ml N-Methylpyrrolidon und 300 ml Wasser 7 Stunden auf 100°C erhitzt. Nach Abdestillieren des N-Methylpyrrolidon/Wasser-Gemisches wird der Rückstand in Toluol suspendiert und die Toluolphase mehrmals mit Wasser gewaschen, um noch vorhandenes N-Methylpyrrolidon zu entfernen. Anschließend wird die Toluolphase filtriert. Man erhält nach Einengen im Vakuum 30,5 g (85,1 %) N-Hydroxyacetyl-(3,5-dimethylanilid) als farblose Kristalle mit einem Schmelzpunkt von 114°C und einem Reingehalt (GC) von 99,3 %.

### Beispiel 5

### Herstellung von Hydroxyessigsäure-(2-methoxyanilid)

In einem 250 ml-Kolben werden 10 g (0,05 mol) Chloressigsäure-(2-methoxyanilid) (hergestellt aus Chloracetylchlorid und 2-Methoxyanilin) mit 6 g (0,55 mol) Natriumcarbonat in einem Lösungsmittelgemisch aus 100 ml N-Methylpyrrolidon und 70 ml Wasser auf 100°C erhitzt. Eine GC-Probe nach 6 Stunden Reaktionszeit ergibt eine Ausbeute von 70 % an Hydroxyessigsäure-(2-methoxyanilid) und von 5,5 % an Diglykolsäure-(2-methoxyanilid).

## Patentansprüche

1. Verfahren zur Herstellung von Hydroxycarbonsäureaniliden der allgemeinen Formel (1) 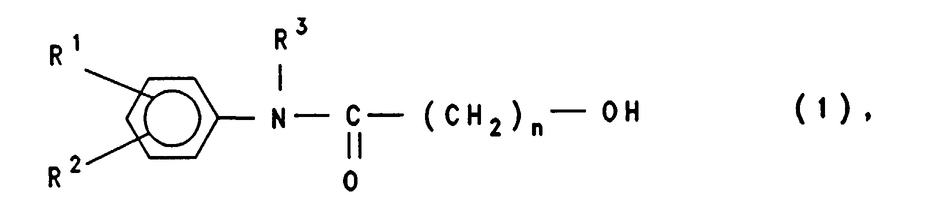 worin R¹ und R² gleich oder verschieden sind und für Wasserstoff, Halogen, eine Nitro-, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, R³ Wasserstoff oder eine geradkettige oder verzweigte Alkyl-Gruppe mit 1 bis 12 Kohlenstoffatomen bedeutet und n für eine ganze Zahl von 1 bis 12 steht, dadurch gekennzeichnet, daß man ein Halogencarbonsäureanilid der allgemeinen Formel (2) 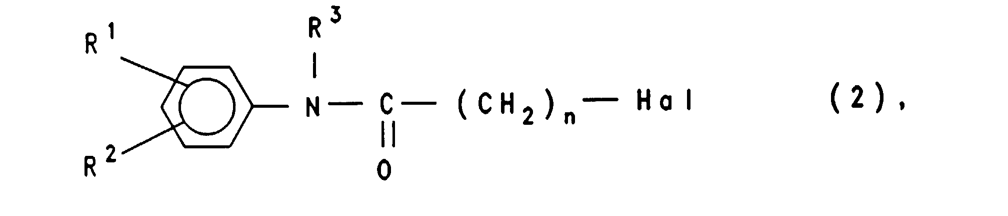 worin R¹, R², R³ und n diesselbe Bedeutung wie in Formel (1) haben und Hal für Chlor, Brom oder Iod steht, mit einer basischen Verbindung in einem Wasser und ein oder mehrere polar aprotische Solventien enthaltenden Lösungsmittelgemisch bei einer Temperatur von 40 bis 180°C umsetzt.

2. Verfahren nach Anspruch 1, dadurch gekennzeichnet, daß man ein Halogencarbonsäureanilid der Formel (2), worin R¹ und R² gleich oder verschieden sind und für Wasserstoff, Halogen, eine Nitro-, eine geradkettige oder verzweigte Alkyl-, Alkoxy-Gruppe mit 1 bis 4 Kohlenstoffatomen, oder eine Aralkyl-Gruppe mit 7 bis 12 Kohlenstoffatomen stehen, einsetzt.

3. Verfahren nach Anspruch 1 oder 2, dadurch gekennzeichnet, daß man ein Halogencarbonsäureanilid der Formel (2), worin R¹ und R² gleich oder verschieden sind und für Wasserstoff, Fluor, Chlor, Brom, eine Alkyl- oder Alkoxy-Gruppe mit 1 bis 4 Kohlenstoffatomen stehen, einsetzt.

4. Verfahren nach einem oder mehreren der Ansprüche 1 bis 3, dadurch gekennzeichnet, daß man ein Halogencarbonsäureanilid der Formel (2), worin R¹ und R² gleich oder verschieden sind und für Wasserstoff, Fluor, Chlor, Brom oder eine Alkylgruppe mit 1 bis 4 Kohlenstoffatomen stehen, einsetzt.

5. Verfahren nach einem oder mehreren der Ansprüche 1 bis 4, dadurch gekennzeichnet, daß man ein Halogencarbonsäureanilid der Formel (2), worin R³ für Wasserstoff oder eine geradkettige oder verzweigte Alkyl-Gruppe mit 1 bis 4 Kohlenstoffatomen steht, einsetzt.

6. Verfahren nach einem oder mehreren der Ansprüche 1 bis 5, dadurch gekennzeichnet, daß man ein Halogencarbonsäureanilid der Formel (2), worin R³ für eine Isopropyl-Gruppe steht, einsetzt.

7. Verfahren nach einem oder mehreren der Ansprüche 1 bis 6, dadurch gekennzeichnet, daß man ein Halogencarbonsäureanilid der Formel (2), worin n für eine ganze Zahl von 1 bis 4 steht, einsetzt.

8. Verfahren nach einem oder mehreren der Ansprüche 1 bis 7, dadurch gekennzeichnet, daß man ein Halogencarbonsäureanilid der Formel (2), worin n für 1 steht, einsetzt.

9. Verfahren nach einem oder mehreren der Ansprüche 1 bis 8, dadurch gekennzeichnet, daß man ein Halogencarbonsäureanilid der Formel (2), worin Hal für Cl oder Br steht, einsetzt.

10. Verfahren nach einem oder mehreren der Ansprüche 1 bis 9, dadurch gekennzeichnet, daß man ein Halogencarbonsäureanilid der Formel (2), worin Hal für Chlor steht, einsetzt.

11. Verfahren nach einem oder mehreren der Ansprüche 1 bis 10, dadurch gekennzeichnet, daß man als basische Verbindung ein Hydroxid, Hydrogencarbonat, Carbonat und/oder Carboxylat eines Alkali- oder Erdalkalimetalls einsetzt.

12. Verfahren nach einem oder mehreren der Ansprüche 1 bis 10, dadurch gekennzeichnet, daß man als basische Verbindung Natriumcarbonat, Kaliumcarbonat oder Natriumacetat einsetzt.

13. Verfahren nach einem oder mehreren der Ansprüche 1 bis 12, dadurch gekennzeichnet, daß man als Lösungsmittelgemisch Wasser und polar aprotisches Solvens im Gewichtsverhältnis 1:4 bis 4:1 einsetzt.

14. Verfahren nach einem oder mehreren der Ansprüche 1 bis 13, dadurch gekennzeichnet, daß man als Lösungsmittelgemisch Wasser und polar aprotisches Solvens im Gewichtsverhältnis 1:2 bis 2:1 einsetzt.

15. Verfahren nach einem oder mehreren der Ansprüche 1 bis 14, dadurch gekennzeichnet, daß man als polar aprotisches Solvens N,N-Dimethylformamid, N,N-Dimethylacetamid, Formamid, Tetrahydrofuran, Dioxan, Dimethylsulfoxid, N-Methylpiperidon oder N-Methylpyrrolidon oder Mischungen derselben einsetzt.

16. Verfahren nach einem oder mehreren der Ansprüche 1 bis 15, dadurch gekennzeichnet, daß man als polar aprotisches Solvens N,N-Dimethylacetamid oder N-Methylpyrrolidon einsetzt.

17. Verfahren nach einem oder mehreren der Ansprüche 1 bis 16, dadurch gekennzeichnet, daß man je Mol Halogencarbonsäureanilid 1 bis 5 Äquivalente der basischen Verbindung einsetzt.

18. Verfahren nach einem oder mehreren der Ansprüche 1 bis 17, dadurch gekennzeichnet, daß man je Mol Halogencarbonsäureanilid 1 bis 1,25 Äquivalente der basischen Verbindung einsetzt.

19. Verfahren nach einem oder mehreren der Ansprüche 1 bis 18, dadurch gekennzeichnet, daß man das Halogencarbonsäureanilid bei einer Temperatur von 60 bis 140°C umsetzt.

20. Verfahren nach einem oder mehreren der Ansprüche 1 bis 19, dadurch gekennzeichnet, daß man das Halogencarbonsäureanilid bei einer Temperatur von 70 bis 120°C umsetzt.

## Claims

1. A process for the preparation of hydroxycarboxanilides of the formula (1) 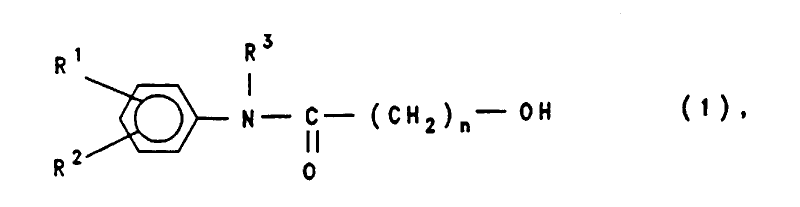 in which R¹ and R² are identical or different and are hydrogen, halogen, a nitro group, a cyano group, a straight-chain or branched alkyl, alkenyl, alkynyl or alkoxy group having 1 to 12 carbon atoms, an aralkyl group having 7 to 12 carbon atoms, a cycloalkyl group having 6 to 12 carbon atoms or an aryl group having 6 to 12 carbon atoms, R³ is hydrogen or a straight-chain or branched alkyl group having 1 to 12 carbon atoms and n is an integer from 1 to 12, which comprises reacting a halocarboxanilide of the formula (2) 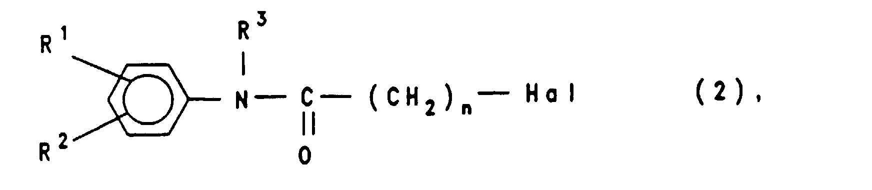 in which R¹, R², R³ and n have the same meaning as in formula (1) and Hal is chlorine, bromine or iodine, with a basic compound in a solvent mixture comprising water and one or more polar aprotic solvents at a temperature of 40 to 180°C.

2. The process as claimed in claim 1, wherein a halocarboxanilide of the formula (2) is employed in which R¹ and R² are identical or different and are hydrogen, halogen, a nitro group, a straight-chain or branched alkyl or alkoxy group having 1 to 4 carbon atoms or an aralkyl group having 7 to 12 carbon atoms.

3. The process as claimed in claim 1 or 2, wherein a halocarboxanilide of the formula (2) is employed in which R¹ and R² are identical or different and are hydrogen, fluorine, chlorine, bromine or an alkyl or alkoxy group having 1 to 4 carbon atoms.

4. The process as claimed in one or more of claims 1 to 3, wherein a halocarboxanilide of the formula (2) is employed in which R¹ and R² are identical or different and are hydrogen, fluorine, chlorine, bromine or an alkyl group having 1 to 4 carbon atoms.

5. The process as claimed in one or more of claims 1 to 4, wherein a halocarboxanilide of the formula (2) is employed in which R³ is hydrogen or a straight-chain or branched alkyl group having 1 to 4 carbon atoms.

6. The process as claimed in one or more of claims 1 to 5, wherein a halocarboxanilide of the formula (2) is employed in which R³ is an isopropyl group.

7. The process as claimed in one of more of claims 1 to 6, wherein a halocarboxanilide of the formula (2) is employed in which n is an integer from 1 to 4.

8. The process as claimed in one of more of claims 1 to 7, wherein a halocarboxanilide of the formula (2) is employed in which n is 1.

9. The process as claimed in one of more of claims 1 to 8, wherein a halocarboxanilide of the formula (2) is employed in which Hal is Cl or Br.

10. The process as claimed in one of more of claims 1 to 9, wherein a halocarboxanilide of the formula (2) is employed in which Hal is chlorine.

11. The process as claimed in one or more of claims 1 to 10, wherein the basic compound employed is a hydroxide, hydrogen carbonate, carbonate and/or carboxylate of an alkali metal or alkaline earth metal.

12. The process as claimed in one or more of claims 1 to 10, wherein the basic compound employed is sodium carbonate, potassium carbonate or sodium acetate.

13. The process as claimed in one or more of claims 1 to 12, wherein the solvent mixture employed is water and polar aprotic solvent in a ratio by weight of 1:4 to 4:1.

14. The process as claimed in one or more of claims 1 to 13, wherein the solvent mixture employed is water and polar aprotic solvent in a ratio by weight of 1:2 to 2:1.

15. The process as claimed in one or more of claims 1 to 14, wherein the polar aprotic solvent employed is N,N-dimethylformamide, N,N-dimethylacetamide, formamide, tetrahydrofuran, dioxane, dimethyl sulfoxide, N-methylpiperidone or N-methylpyrrolidone, or mixtures of these.

16. The process as claimed in one or more of claims 1 to 15, wherein the polar aprotic solvent employed is N,N-dimethylacetamide or N-methylpyrrolidone.

17. The process as claimed in one or more of claims 1 to 16, wherein 1 to 5 equivalents of the basic compound are employed per mole of halocarboxanilide.

18. The process as claimed in one or more of claims 1 to 17, wherein 1 to 1.25 equivalents of the basic compound are employed per mole of halocarboxanilide.

19. The process as claimed in one or more of claims 1 to 18, wherein the halocarboxanilide is reacted at a temperature from 60 to 140°C.

20. The process as claimed in one or more of claims 1 to 19, wherein the halocarboxanilide is reacted at a temperature from 70 to 120°C.

## Revendications

1. Procédé de préparation d'anilides d'acides hydroxycarboxyliques de formule générale (1) 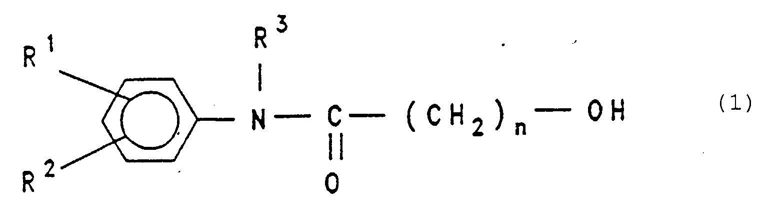 où R¹ et R² sont identiques ou différents et représentent un atome d'hydrogène, un atome d'halogène, un groupe nitro, cyano, alkyle, alcényle, alcynyle, alcoxy avec 1 à 12 atomes de carbone, à chaîne linéaire ou ramifiée, un groupe aralkyle avec 7 à 12 atomes de carbone, un groupe cycloalkyle avec 6 à 12 atomes de carbone ou un groupe aryle avec 6 à 12 atomes de carbone, R³ représente un atome d'hydrogène ou un groupe alkyle avec 1 à 12 atomes de carbone, à chaîne linéaire ou ramifiée, et n est un entier 1 à 12, caractérisé en ce que l'on fait réagir un anilide d'acide halogénocarboxylique de formule générale (2) 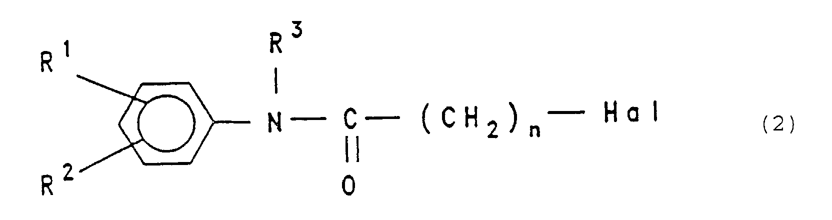 où R¹, R², R³ et n ont la même signification que dans la formule (1) et Hal représente un atome de chlore, de brome ou d'iode, avec un composé basique dans un mélange de solvants contenant de l'eau et un ou plusieurs solvants polaires aprotiques, à une température de 40 °C à 180 °C.

2. Procédé selon la revendication 1, caractérisé en ce que l'on utilise un anilide d'acide halogénocarboxylique de formule (2), où R¹ et R² sont identiques ou différents et représentent un atome d'hydrogène, un atome d'halogène, un groupe nitro, un groupe alkyle, un groupe alcoxy avec 1 à 4 atomes de carbone linéaire ou ramifié, ou un groupe aralkyle avec 7 à 12 atomes de carbone.

3. Procédé selon la revendication 1 ou 2, caractérisé en ce que l'on utilise un anilide d'acide halogénocarboxylique de formule (2), où R¹ et R² sont identiques ou différents et représentent un atome d'hydrogène, de fluor, de chlore, de brome, un groupe alkyle ou alcoxy avec 1 à 4 atomes de carbone.

4. Procédé selon une ou plusieurs des revendications 1 à 3, caractérisé en ce que l'on utilise un anilide d'acide halogénocarboxylique de formule (2), où R¹ et R² sont identiques ou différents et représentent un atome d'hydrogène, un atome de fluor, de chlore, de brome ou un groupe alkyle avec 1 à 4 atomes de carbone.

5. Procédé selon une ou plusieurs des revendications 1 à 4, caractérisé en ce que l'on utilise un anilide d'acide halogénocarboxylique de formule (2) , où R³ représente un atome d'hydrogène ou un groupe alkyle avec 1 à 4 atomes de carbone à chaîne linéaire ou ramifiée.

6. Procédé selon une ou plusieurs des revendications 1 à 5, caractérisé en ce que l'on utilise un anilide d'acide halogénocarboxylique de formule (2), où R³ représente un groupe isopropyle.

7. Procédé selon une ou plusieurs des revendications 1 à 6, caractérisé en ce que l'on utilise un anilide d'acide halogénocarboxylique de formule (2), où n est un nombre entier de 1 à 4.

8. Procédé selon une ou plusieurs des revendications 1 à 7, caractérisé en ce que l'on utilise un anilide d'acide halogénocarboxylique de formule (2), où n vaut 1.

9. Procédé selon une ou plusieurs des revendications 1 à 8, caractérisé en ce que l'on utilise un anilide d'acide halogénocarboxylique de formule (2), où Hal représente un atome de chlore ou de brome.

10. Procédé selon une ou plusieurs des revendications 1 à 9, caractérisé en ce que l'on utilise un anilide d'acide halogénocarboxylique de formule (2), où Hal représente un atome de chlore.

11. Procédé selon l'une quelconque des revendications 1 à 10, caractérisé en ce que l'on utilise comme composé basique un hydroxyde, un bicarbonate, un carbonate et/ou carboxylate de métaux alcalins ou alcalino-terreux.

12. Procédé selon une ou plusieurs des revendications 1 à 10, caractérisé en ce que l'on utilise comme composé basique le carbonate de sodium, le carbonate de potassium ou l'acétate de sodium.

13. Procédé selon une ou plusieurs des revendications 1 à 12, caractérisé en ce que l'on utilise comme mélange de solvants l'eau et des solvants polaires aprotiques dans un rapport de 1:4 à 4:1.

14. Procédé selon une ou plusieurs des revendications 1 à 13, caractérisé en ce que l'on utilise comme mélange de solvants l'eau et des solvants polaires aprotiques dans un rapport de 1:2 à 2:1.

15. Procédé selon une ou plusieurs des revendications 1 à 14, caractérisé en ce que l'on utilise comme solvant aprotique le N,N-diméthylformamide, le N,N-diméthylacétamide, formamide, le tétrahydrofuranne, le dioxanne, le diméthylsulfoxyde, la N-méthylpipéridone ou la N-méthylpyrrolidone ou leurs mélanges.

16. Procédé selon une ou plusieurs des revendications 1 à 15, caractérisé en ce que l'on utilise comme solvant aprotique polaire le N,N-diméthylacétamide ou la N-méthylpyrrolidone.

17. Procédé selon une ou plusieurs des revendications 1 à 16, caractérisé en ce que l'on utilise pour chaque mole d'anilide d'acide halogénocarboxylique de 1 à 5 équivalents de composé basique.

18. Procédé selon une plusieurs des revendications 1 à 17, caractérisé en ce que l'on utilise pour chaque mole d'anilide d'acide halogénocarboxylique de 1 à 1,25 équivalents de composé basique.

19. Procédé selon une ou plusieurs des revendications 1 à 18, caractérisé en ce que l'on fait réagir l'anilide d'acide halogénocarboxylique à une température de 60 °C à 140 °C.

20. Procédé selon une ou plusieurs des revendications 1 à 19, caractérisé en ce que l'on fait réagir l'anilide d'acide halogénocarboxylique à une température de 70 °C à 120 °C.
